# EUROPEAN PATENT APPLICATION

(11) **EP 4 434 557 A1**
(43) Date of publication of application: **25.09.2024**
(21) Application number: 23198679.5
(22) Date of filing: 21.09.2023
(51) Int. Cl.: A61M 5/145, A61M 5/32, A61M 5/42, A61M 5/44, A61M 5/46, A61F 7/00

(54) **CONVEYING LOCAL CRYOANESTHESIA TYPE PAINLESS SYRINGE**

(30) Priority: 24.03.2023 KR 20230038327
(71) Applicant: Woorhimecha Co., Ltd., Gunpo-si, Gyeonggi-do (KR)
(72) Inventor: Woo, Yong Woon, Seoul (KR)
(74) Representative: Gulde & Partner

(57) **Abstract**

The present disclosure relates to a device for conveying a local cryoanesthesia type painless syringe, and more particularly, to a device for conveying a local cryoanesthesia type painless syringe, the device having a first needle guide hole formed at the center of a skin anesthetizing plate, which is mounted on one side of a device body to come in contact with the skin, to instantaneously cool the epidermis and block neural transmission, a second needle guide hole formed at the center of a Peltier element, which is in close contact with a rear side surface of the skin anesthetizing plate, to absorb heat generated from the skin anesthetizing plate, a third needle guide hole formed at the center of a heat dissipation block, which connects a lower side thereof with a second heat dissipation plate having a cooling fan, positioned at a lower side of the device body, attached thereto using a heat pipe, to absorb heat from the Peltier element and dissipate heat, a fourth needle guide hole formed at the center of a first heat dissipation plate, which is in close contact with a rear side surface of the heat dissipation block, to absorb heat of the heat dissipation block and dissipate heat to the outside, a support clip, configured to loosely clamp one side of a syringe cylinder, formed at an upper side of a moving stand to allow a syringe needle coupled to the syringe cylinder to be positioned on a center line of the fourth, third, second, and first needle guide holes, a moving stand conveyor configured to move the moving stand toward the skin to convey the syringe cylinder so that the syringe needle of the syringe cylinder is able to pass through the fourth, third, second, and first needle guide holes and be inserted at a predetermined depth into the skin, a moving clip in which an insertion groove is formed to, unlike the support clip of the moving stand, tightly clamp a flange fitted thereinto formed at a rear side of the syringe cylinder, and a moving clip position controller configured to, corresponding to a short length (b) or a long length (c) of the syringe needle coupled to the syringe cylinder that remains fixed to the moving clip, place an end of the syringe needle at a set point (a).

## Description

### CROSS-REFERENCE TO RELATED APPLICATION

This application claims priority to and the benefit of Korean Patent Application No. 10-2023-0038327, filed on March 24, 2023, the disclosure of which is incorporated herein by reference in its entirety.

### BACKGROUND

### 1. Field of the Invention

The present disclosure relates to a device for conveying a local cryoanesthesia type painless syringe, and more particularly, to a device for conveying a local cryoanesthesia type painless syringe, the device having a first needle guide hole formed at the center of a skin anesthetizing plate, which is mounted on one side of a device body to come in contact with the skin, to instantaneously cool the epidermis and block neural transmission, a second needle guide hole formed at the center of a Peltier element, which is in close contact with a rear side surface of the skin anesthetizing plate, to absorb heat generated from the skin anesthetizing plate, a third needle guide hole formed at the center of a heat dissipation block, which connects a lower side thereof with a second heat dissipation plate having a cooling fan, positioned at a lower side of the device body, attached thereto using a heat pipe, to absorb heat from the Peltier element and dissipate heat, a fourth needle guide hole formed at the center of a first heat dissipation plate, which is in close contact with a rear side surface of the heat dissipation block, to absorb heat of the heat dissipation block and dissipate heat to the outside, a moving stand having a support clip, configured to loosely clamp one side of a syringe cylinder, formed at an upper side to allow a syringe needle coupled to the syringe cylinder to be positioned on a center line of the fourth, third, second, and first needle guide holes, a moving stand conveyor configured to move the moving stand toward the skin to convey the syringe cylinder so that the syringe needle of the syringe cylinder is able to pass through the fourth, third, second, and first needle guide holes and be inserted at a predetermined depth into the skin, a moving clip in which an insertion groove is formed to, unlike the support clip of the moving stand, tightly clamp a flange fitted thereinto formed at a rear side of the syringe cylinder, and a moving clip position controller configured to, corresponding to a short length (b) or a long length (c) of the syringe needle coupled to the syringe cylinder that remains fixed to the moving clip, place an end of the syringe needle at a set point (a), wherein, due to the result that, when a controlling handle is rotated and reversely rotated, a controlling screw shaft is rotated and reversely rotated and moves the moving clip toward one side and the other side, it is possible to move the syringe cylinder having the flange inserted into the insertion groove of the moving clip, and thus, regardless of the short length (b) or the long length (c) of the syringe needle coupled to the syringe cylinder, the end of the syringe needle can be placed at the set point (a) and a phenomenon in which the syringe needle is inserted too shallow or too deep into the skin can be prevented, thereby creating conditions that allow the syringe needle to be inserted at the same appropriate depth in a more stable state, and due to the result that, when a start button of the device body is pressed, a conveying screw shaft inside the device body rotates due to an operation of a driving motor and moves a connecting stand and a moving stand coupled to the conveying screw shaft toward the skin, the syringe needle can be accurately inserted at a set depth into the skin, thus reaching a stable preliminary state in which an injection can be injected, and, after the syringe needle is inserted into the skin, a person who injects an injection (a doctor or a nurse) can directly press a syringe piston to inject an appropriate amount of the injection and inject a desired amount of the injection, thus allowing the injection to be accurately injected in an amount suitable for a patient.

### 2. Discussion of Related Art

As the related art, Korean Patent Registration No. 10-0851274 "Probe for local anesthetic system" has been registered after being filed as an application.

In the related art, a probe for a local anesthetic system for cooling a local site that requires anesthesia includes: a cooling pad made of a thermally conductive material and having a contact surface of a predetermined area formed to come in contact with an anesthetic site; a liquid-phase heat capacity adding means configured to increase the heat capacity of the cooling pad; and a thermoelectric element installed to emit cold air to at least any one of the heat capacity adding means and the cooling pad.

In the related art, since an anesthetic action is performed by instantaneously cooling the epidermis and blocking neural transmission, anesthesia can be conveniently performed without causing pain, and in particular, since the heat capacity of the cooling pad is increased by the heat capacity adding means, anesthesia can be stably performed repeatedly.

However, the related art only discloses performing local cryoanesthesia to eliminate pain and does not disclose a technology for adjusting the position of a syringe needle of a painless syringe or a technology for moving a syringe needle to insert the syringe needle into the skin, which is disclosed in the present disclosure. Therefore, the related art is significantly different from the present disclosure in terms of the objectives, configurations, effects, and the like.

### SUMMARY OF THE INVENTION

The present disclosure is directed to providing a device for conveying a local cryoanesthesia type painless syringe in which, due to the result that, when a controlling handle is rotated and reversely rotated, a controlling screw shaft is rotated and reversely rotated and moves a moving clip toward one side and the other side, it is possible to move a syringe cylinder having a flange inserted into an insertion groove of the moving clip, and thus, regardless of a short length (b) or a long length (c) of a syringe needle coupled to the syringe cylinder, an end of the syringe needle can be placed at a set point (a) and a phenomenon in which the syringe needle is inserted too shallow or too deep into the skin can be prevented, thereby creating conditions that allow the syringe needle to be inserted at the same appropriate depth in a more stable state, and due to the result that, when a start button of a device body is pressed, a conveying screw shaft inside the device body rotates due to an operation of a driving motor and moves a connecting stand and a moving stand coupled to the conveying screw shaft toward the skin, the syringe needle can be accurately inserted at a set depth into the skin, thus reaching a stable preliminary state in which an injection can be injected, and, after the syringe needle is inserted into the skin, a person who injects an injection (a doctor or a nurse) can directly press a syringe piston to inject an appropriate amount of the injection and inject a desired amount of the injection, thus allowing the injection to be accurately injected in an amount suitable for a patient.

The present disclosure provides a device for conveying a local cryoanesthesia type painless syringe, the device having: a first needle guide hole formed at the center of a skin anesthetizing plate, which is mounted on one side of a device body to come in contact with the skin, to instantaneously cool the epidermis and block neural transmission; a second needle guide hole formed at the center of a Peltier element, which is in close contact with a rear side surface of the skin anesthetizing plate, to absorb heat generated from the skin anesthetizing plate; a third needle guide hole formed at the center of a heat dissipation block, which connects a lower side thereof with a second heat dissipation plate having a cooling fan, positioned at a lower side of the device body, attached thereto using a heat pipe, to absorb heat from the Peltier element and dissipate heat; a fourth needle guide hole formed at the center of a first heat dissipation plate, which is in close contact with a rear side surface of the heat dissipation block, to absorb heat of the heat dissipation block and dissipate heat to the outside; a moving stand having a support clip, configured to loosely clamp one side of a syringe cylinder, formed at an upper side to allow a syringe needle coupled to the syringe cylinder to be positioned on a center line of the fourth, third, second, and first needle guide holes; a moving stand conveyor configured to move the moving stand toward the skin to convey the syringe cylinder so that the syringe needle of the syringe cylinder is able to pass through the fourth, third, second, and first needle guide holes and be inserted at a predetermined depth into the skin; a moving clip in which an insertion groove is formed to, unlike the support clip of the moving stand, tightly clamp a flange fitted thereinto formed at a rear side of the syringe cylinder; and a moving clip position controller configured to, corresponding to a short length (b) or a long length (c) of the syringe needle coupled to the syringe cylinder that remains fixed to the moving clip, place an end of the syringe needle at a set point (a).

### BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:
FIG 1 is a perspective view illustrating a configuration of a device for conveying a painless syringe according to the present disclosure;
FIG 2 is an action cross-sectional view for describing the configuration and action of the device for conveying a painless syringe according to the present disclosure;
FIGS. 3 to 5 are action views for describing a syringe needle position adjusting action of the device for conveying a painless syringe according to the present disclosure; and
FIG 6 is an action cross-sectional view for describing a syringe needle conveying action of the device for conveying a painless syringe according to the present disclosure.

### DETAILED DESCRIPTION OF EXEMPLARY EMBODIMENTS

The configuration of the present disclosure will be described in detail with reference to the accompanying drawings.

A device for conveying a local cryoanesthesia type painless syringe includes: a first needle guide hole 132 formed at the center of a skin anesthetizing plate 131, which is mounted on one side of a device body 101 to come in contact with the skin, to instantaneously cool the epidermis and block neural transmission; a second needle guide hole 134 formed at the center of a Peltier element 133, which is in close contact with a rear side surface of the skin anesthetizing plate 131, to absorb heat generated from the skin anesthetizing plate 131; a third needle guide hole 136 formed at the center of a heat dissipation block 135, which connects a lower side thereof with a second heat dissipation plate 142 having a cooling fan 143, positioned at a lower side of the device body 101, attached thereto using a heat pipe 141, to absorb heat from the Peltier element 133 and dissipate heat; a fourth needle guide hole 138 formed at the center of a first heat dissipation plate 137, which is in close contact with a rear side surface of the heat dissipation block 135, to absorb heat of the heat dissipation block 135 and dissipate heat to the outside; a moving stand 116 having a support clip 117, configured to loosely clamp one side of a syringe cylinder 151, formed at an upper side to allow a syringe needle 154 coupled to the syringe cylinder 151 to be positioned on a center line of the fourth, third, second, and first needle guide holes 138, 136, 134, and 132; a moving stand conveyor configured to move the moving stand 116 toward the skin to convey the syringe cylinder 151 so that the syringe needle 154 of the syringe cylinder 151 is able to pass through the fourth, third, second, and first needle guide holes 138, 136, 134, and 132 and be inserted at a predetermined depth into the skin; a moving clip 125 in which an insertion groove 126 is formed to, unlike the support clip 117 of the moving stand 116, tightly clamp a flange 152 fitted thereinto formed at a rear side of the syringe cylinder 151; and a moving clip position controller configured to, corresponding to a short length b or a long length c of the syringe needle 154 coupled to the syringe cylinder 151 that remains fixed to the moving clip 125, place an end of the syringe needle 154 at a set point a.

Also, the moving stand conveyor includes: a guide rail 104 formed on an inner side surface of the device body 101 and configured to guide movement of the moving stand 116 while supporting a bottom portion of the moving stand 116 so that the moving stand 116 is able to move in a stable state; a connecting stand 115 on which a first internal screw portion 114 coupled to a central lower side of the moving stand 116 is formed; a conveying screw shaft 113 fastened to the first internal screw portion 114 of the connecting stand 115 and rotatably coupled to one side and the other side inside the device body 101; and a driving motor 111 mounted on a bottom portion of one side of the device body 101, connecting one end of the conveying screw shaft 113 with a driving shaft 112, to rotate and reversely rotate the conveying screw shaft 113.

Also, the moving clip position controller includes: a connecting piece 124 on which a second internal screw portion 123 attached to a lower side of the moving clip 125 is formed; a controlling screw shaft 122 fastened to the second internal screw portion 123 of the connecting piece 124 and rotatably coupled to one side and the other side inside the moving stand 116; and a controlling handle 121 connected to the other side of the controlling screw shaft 122 and exposed to the outside to rotate and reversely rotate the controlling screw shaft 122.

The action of the present disclosure will be described below.

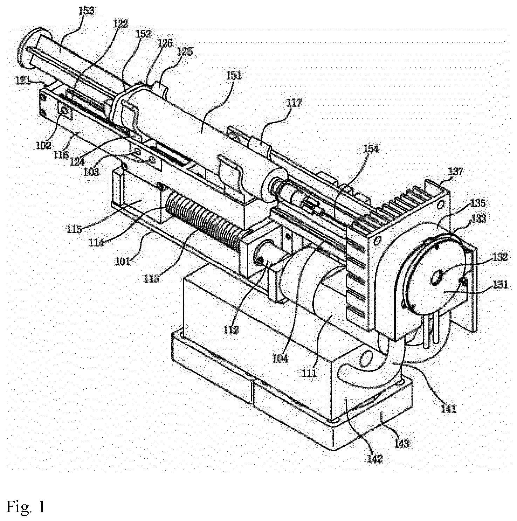

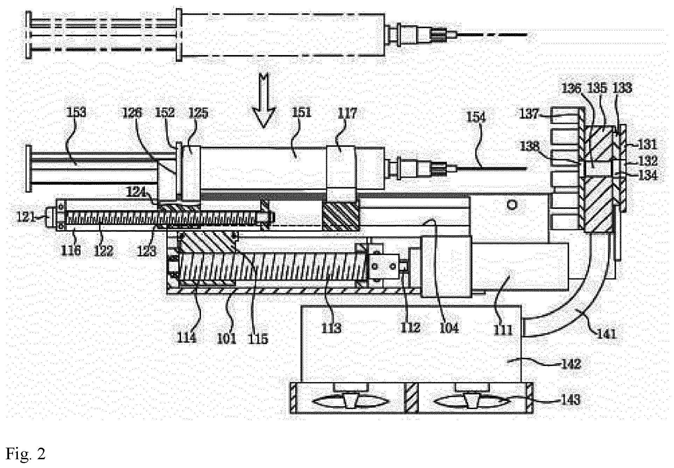

FIG 1 is a perspective view illustrating the configuration of the device for conveying a painless syringe according to the present disclosure, and FIG 2 is an action cross-sectional view for describing the configuration and action of the device for conveying a painless syringe according to the present disclosure. The syringe cylinder 151 is filled with an injection to be injected into a patient, and then a syringe piston 153 is inserted into the syringe cylinder 151.

Also, one side of the syringe cylinder 151 holding the injection is loosely fitted to and supported by the support clip 117 coupled to an upper portion of one side of the moving stand 116 placed on the guide rail 104 of the device body 101, and then the flange 152 formed at the rear side of the syringe cylinder 151 is firmly inserted into and fixed to the insertion groove 126 of the moving clip 125 formed on an upper side of the connecting piece 124 positioned inside the moving stand.

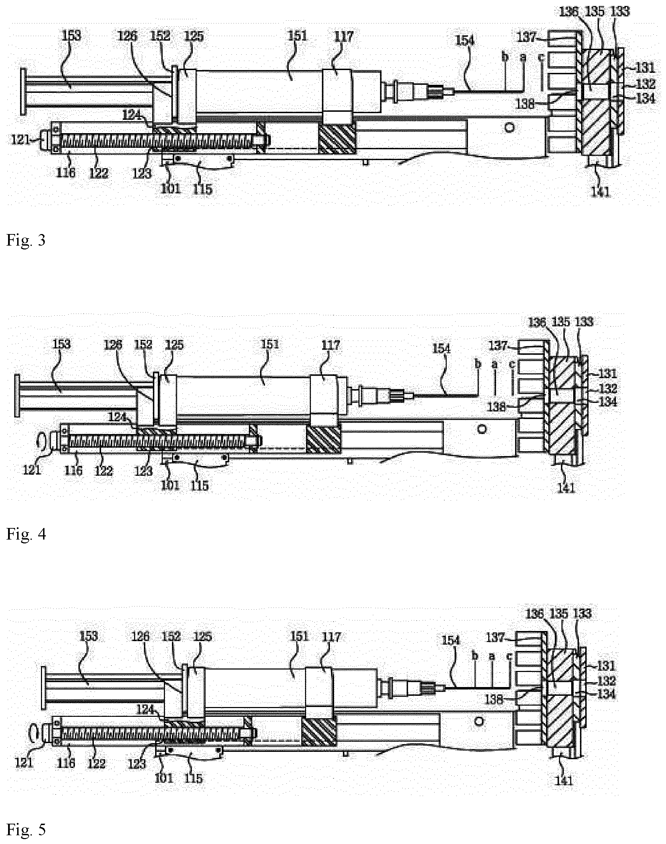

Once the syringe cylinder 151 is completely seated on the moving clip 125 and the support clip 117 as described above, when the end of the syringe needle 154 coupled to one side of the syringe cylinder 151 is positioned at point "a," which is an initial point, as illustrated in FIG 3, a subsequent operation is performed without any problem. However, the end of the syringe needle 154 is positioned at point "b," which makes the length of the syringe needle 154 short, or point "c," which makes the length of the syringe needle 154 long, in some cases.

In such cases, when the controlling handle 121 coupled to a rear side of the moving stand 116 is rotated and reversely rotated, the controlling screw shaft 122 inside the moving stand 116 connected to the controlling handle 121 is rotated and reversely rotated and allows movement of the connecting piece 124 and the moving clip 125, which fasten the second internal screw portion 123 to the controlling screw shaft 122, toward one side and the other side.

Accordingly, since the syringe cylinder 151 having the flange 152 inserted into the insertion groove 126 of the moving clip 125 moves toward one side and the other side, the support clip 117 fixed to one side of the moving stand 116 does not receive resistance against the support clip 117 due to loosely coupling one side of the syringe cylinder 151, and thus it is possible to place the end of the syringe needle 154 positioned at point "b" as illustrated in FIG 4 and the end of the syringe needle 154 positioned at point "c" as illustrated in FIG 5 at point "a" illustrated in FIG 4, which is the initial point.

When the end of the syringe needle 154 is positioned at point "a" which is the initial point, the point can be checked as a bell rings due to a detecting action of a control box (not illustrated).

When, as described above, the end of the syringe needle 154 reaches point "a" which is the initial position, a person who injects an injection waits until the color of light emitted by an indicator lamp 103 coupled to one side surface of the device body 101 turns from red to green.

That is, when the indicator lamp 103 is emitting red light, since the temperature of the skin anesthetizing plate 131 coupled to one side of the device body 101 is dropping due to the action of the Peltier element 133, heat dissipated from the Peltier element 133 is dissipated to the outside through the heat dissipation block 135 and the first heat dissipation plate 137, and heat dissipated from the second heat dissipation plate 142, connected to the lower side of the heat dissipation block 135 using the heat pipe 141, is dissipated to the outside by the cooling fan 143.

When the temperature of the skin anesthetizing plate 131 drops to an appropriate temperature due to the Peltier element 133 as described above, the color of light emitted by the indicator lamp 103 of the device body 101 turns green.

When the indicator lamp 103 is emitting green light, the person who injects an injection brings the skin anesthetizing plate 131 in contact with the patient's skin to perform cryoanesthesia thereon and then presses a start button 102 coupled to the device body 101 to operate the driving motor 111 mounted on a lower side of one side of the device body 101. In this way, the driving shaft 112 is driven, and the conveying screw shaft 113, disposed inside the device body 101 and having one side connected to the driving shaft 112, rotates.

Also, due to the rotation of the conveying screw shaft 113, the connecting stand 115 fastening the first internal screw portion 114 to the conveying screw shaft 113, the moving stand 116 connected to an upper side of the connecting stand 115, and the moving clip 125 and the support clip 117 which are positioned at an upper side of the moving stand 116 are conveyed toward the skin together with the syringe cylinder 151.

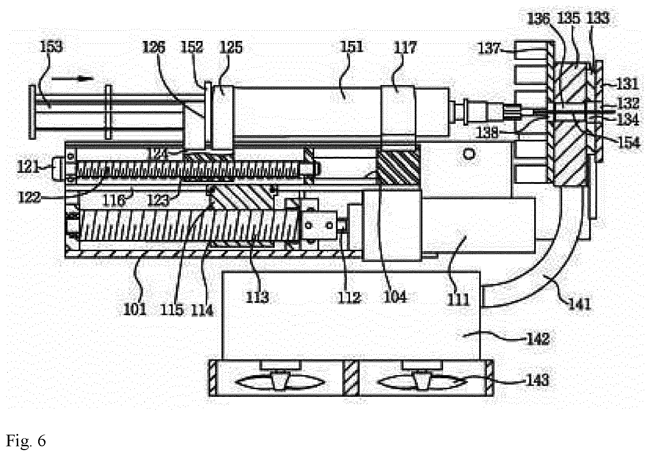

Also, due to the syringe cylinder 151 being conveyed, the syringe needle 154 coupled to one side of the syringe cylinder 151 passes through the fourth needle guide hole 138 formed at the center of the first heat dissipation plate 137, the third needle guide hole 136 formed at the center of the heat dissipation block 135, the second needle guide hole 134 formed at the center of the Peltier element 133, and the first needle guide hole 132 formed at the center of the skin anesthetizing plate 131 and is inserted at a predetermined depth into the skin as illustrated in FIG 6.

When the syringe needle 154 is inserted into the skin as described above, the person who injects an injection (a doctor or a nurse) presses the syringe piston 153 coupled to a rear side of the syringe cylinder 151 to inject a predetermined amount of the injection.

When the injection is completed as described above, the start button 102 is pressed again. In this case, since the driving motor 111 is reversely rotated, thus causing the conveying screw shaft 113 to reversely rotate and the connecting stand 115 fastened to the conveying screw shaft 113 to move toward the other side, the moving stand 116, the moving clip 125, the support clip 117, and the syringe cylinder 151 all return to their original positions illustrated in FIG 2 and wait for a subsequent operation.

According to the present disclosure, due to the result that, when a controlling handle is rotated and reversely rotated, a controlling screw shaft is rotated and reversely rotated and moves a moving clip toward one side and the other side, it is possible to move a syringe cylinder having a flange inserted into an insertion groove of the moving clip, and thus, regardless of a short length (b) or a long length (c) of a syringe needle coupled to the syringe cylinder, an end of the syringe needle can be placed at a set point (a) and a phenomenon in which the syringe needle is inserted too shallow or too deep into the skin can be prevented, thereby creating conditions that allow the syringe needle to be inserted at the same appropriate depth in a more stable state.

According to the present disclosure, due to the result that, when a start button of a device body is pressed, a conveying screw shaft inside the device body rotates due to an operation of a driving motor and moves a connecting stand and a moving stand coupled to the conveying screw shaft toward the skin, the syringe needle can be accurately inserted at a set depth into the skin, thus reaching a stable preliminary state in which an injection can be injected.

According to the present disclosure, after the syringe needle is inserted into the skin, a person who injects an injection (a doctor or a nurse) can directly press a syringe piston to inject an appropriate amount of the injection and inject a desired amount of the injection, thus allowing the injection to be accurately injected in an amount suitable for a patient.

## Claims

1. A device for conveying a local cryoanesthesia type painless syringe, the device comprising:
a first needle guide hole (132) formed at a center of a skin anesthetizing plate (131), which is mounted on one side of a device body (101) to come in contact with the skin, to instantaneously cool the epidermis and block neural transmission;
a second needle guide hole (134) formed at a center of a Peltier element (133), which is in close contact with a rear side surface of the skin anesthetizing plate (131), to absorb heat generated from the skin anesthetizing plate (131);
a third needle guide hole (136) formed at a center of a heat dissipation block (135), which connects a lower side thereof with a second heat dissipation plate (142) having a cooling fan (143), positioned at a lower side of the device body (101), attached thereto using a heat pipe (141), to absorb heat from the Peltier element (133) and dissipate heat;
a fourth needle guide hole (138) formed at a center of a first heat dissipation plate (137), which is in close contact with a rear side surface of the heat dissipation block (135), to absorb heat of the heat dissipation block (135) and dissipate heat to the outside;
a moving stand (116) having a support clip (117), configured to loosely clamp one side of a syringe cylinder (151), formed at an upper side to allow a syringe needle (154) coupled to the syringe cylinder (151) to be positioned on a center line of the first, second, third, and fourth needle guide holes (132) (134) (136) (138);
a moving stand conveyor configured to move the moving stand (116) toward the skin to convey the syringe cylinder (151) so that the syringe needle (154) of the syringe cylinder (151) is able to pass through the first, second, third, and fourth needle guide holes (132) (134) (136) (138) and be inserted at a predetermined depth into the skin;
a moving clip (125) in which an insertion groove (126) is formed to, unlike the support clip (117) of the moving stand (116), tightly clamp a flange (152) fitted thereinto formed at a rear side of the syringe cylinder (151); and
a moving clip position controller configured to, corresponding to a short length (b) or a long length (c) of the syringe needle (154) coupled to the syringe cylinder (151) that remains fixed to the moving clip (125), place an end of the syringe needle (154) at a set point (a).

2. The device of claim 1, wherein the moving stand conveyor includes:
a guide rail (104) formed on an inner side surface of the device body (101) and configured to guide movement of the moving stand (116) while supporting a bottom portion of the moving stand (116) so that the moving stand (116) is able to move in a stable state;
a connecting stand (115) on which a first internal screw portion (114) coupled to a central lower side of the moving stand (116) is formed;
a conveying screw shaft (113) fastened to the first internal screw portion (114) of the connecting stand (115) and rotatably coupled to one side and the other side inside the device body (101); and
a driving motor (111) mounted on a bottom portion of one side of the device body (101), connecting one end of the conveying screw shaft (113) with a driving shaft (112), to rotate and reversely rotate the conveying screw shaft (113).

3. The device of claim 1, wherein the moving clip position controller includes:
a connecting piece (124) on which a second internal screw portion (123) attached to a lower side of the moving clip (125) is formed;
a controlling screw shaft (122) fastened to the second internal screw portion (123) of the connecting piece (124) and rotatably coupled to one side and the other side inside the moving stand (116); and
a controlling handle (121) connected to the other side of the controlling screw shaft (122) and exposed to the outside to rotate and reversely rotate the controlling screw shaft (122).
